(19) Europäisches Patentamt
European Patent Office
Office européen des brevets

(11) EP 0 543 739 B1

(12) # FASCICULE DE BREVET EUROPEEN

(45) Date de publication et mention de la délivrance du brevet: **31.01.1996 Bulletin 1996/05**

(51) Int. Cl.$^6$: **C07C 67/38**, C07C 69/44

(21) Numéro de dépôt: **92420397.9**

(22) Date de dépôt: **05.11.1992**

(54) **Procédé de préparation d'adipates d'alkyle**

Verfahren zur Herstellung von Alkyl-Adipaten

Process for the preparation of alkyl adipates

(84) Etats contractants désignés:
**BE DE ES FR GB IT NL PT**

(30) Priorité: **21.11.1991 FR 9114605**

(43) Date de publication de la demande:
**26.05.1993 Bulletin 1993/21**

(73) Titulaire: **RHONE-POULENC CHIMIE**
**F-92408 Courbevoie Cédex (FR)**

(72) Inventeurs:
- **Denis, Philippe**
  **F-69150 Decines (FR)**
- **Grosselin, Jean-Michel**
  **F-69340 Francheville (FR)**
- **Jenck, Jean**
  **F-69680 Chassieu (FR)**
- **Metz, François**
  **F-69390 Vernaison (FR)**
- **Rouyer, Paul**
  **F-69001 Lyon (FR)**

(74) Mandataire: **Vignally, Noel et al**
**F-69192 Saint-Fons Cédex (FR)**

(56) Documents cités:
**EP-A- 0 080 957** **EP-A- 0 301 450**
**FR-A- 2 384 738**

- **BULLETIN OF THE CHEMICAL SOCIETY OF JAPAN vol. 46, 1973, pages 524 - 530 A. MATSUDA 'The cobalt carbonyl-catalyzed hydroesterification of butadiene with carbon monoxide and methanol'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.9.8/3.4

## Description

La présente invention concerne un procédé de préparation d'adipates d'alkyle par réaction de l'oxyde de carbone et d'un alcool sur un mélange de pentènoates d'alkyle.

Il est bien connu, d'après le "Bulletin of Chemical Society of Japan, vol. 46, 1973, p. 524-530" qu'on obtient un mélange renfermant des diesters d'alkyle et notamment, de l'adipate d'alkyle, en faisant réagir de l'oxyde de carbone et un alcool sur un pentène-3 oate d'alkyle, sous haute pression et à température élevée, en présence de cobalt carbonyle et d'une base azotée hétérocyclique, par exemple la pyridine.

Il est également bien connu, d'après cette même référence qu'on obtient un pentène-3-oate d'alkyle en faisant réagir de l'oxyde de carbone et un alcool sur du butadiène, sous haute pression et à température élevée, en présence de cobalt carbonyle et d'une base azotée hétérocyclique, par exemple la pyridine.

Il a été proposé ( cf. le brevet français n° 2 356 622) de préparer des pentène-3 oates d'alkyle en faisant réagir de l'oxyde de carbone et un alcool sur du butadiène contenu dans une coupe $C_4$ sous une pression d'oxyde de carbone de 300 à 1000 bar et à une température de 100 à 160°C en présence d'un système catalytique à base de cobalt et d'une base azotée hétérocyclique, telle la pyridine et les bases apparentées. Le rendement en pentène-3 oate de méthyle est de l'ordre de 95 %. De nombreuses propositions se sont succédées visant entre autres, soit à améliorer les performances (activité et/ou, sélectivité) de la réaction de conversion du pentène-3 oate d'alkyle en adipate d'alkyle soit à faciliter le recyclage du catalyseur à base de cobalt dans l'une comme dans l'autre des deux réactions en cause, soit à récupérer l'adipate recherché dans le produit brut de la seconde réaction, soit à adoucir les conditions de pression de la première réaction en cause. A titre d'exemples de ces diverses propositions il convient de rappeler :

- le procédé décrit dans le brevet français n° 2 384 738, procédé comprenant une première étape lors de laquelle le butadiène est converti en pentène-3 oate d'alkyle dans des conditions similaires à celle précédemment évoquées, suivie de la séparation du milieu réactionnel d'une partie des bases azotées tertiaires et des hydrocarbures en excès, et une seconde étape lors de laquelle le pentène-3 oate d'alkyle restant dans le mélange réactionnel est converti en un mélange de diesters dans lequel prédomine l'adipate d'alkyle. La première étape est réalisée à des températures comprises entre 80 et 150°C sous des pressions de 300 à 2000 bars ; la seconde étape est réalisée à des températures comprises entre 140 et 200°C, sous des pressions de 100 à 400 bar ;
- le procédé décrit dans le brevet européen n° 60734 proposant de réaliser ladite première étape en présence d'un catalyseur à base de palladium ;
- le procédé décrit dans la demande européenne n° 192 340 proposant d'augmenter la concentration en cobalt dans ladite première étape, afin de pouvoir diminuer sensiblement la pression requise pour son déroulement satisfaisant, et d'enchaîner, sans éliminer de constituant du milieu réactionnel issu de cette première étape, ladite seconde étape ;
- le procédé décrit dans le brevet européen n° 80 957 proposant notamment de réaliser la seconde étape au départ d'un pentène-2 oate d'alkyle et
- le procédé décrit dans la demande de brevet européen n° 177641 proposant de régénérer le catalyseur désactivé, à base de cobalt, par mise en contact d'un mélange le renfermant avec une résine échangeuse d'ions fortement acide, avant son recyclage en réaction.

Le traitement proposé dans ce dernier document mentionné aurait une double fonction, à savoir :

- éliminer les ions N-méthylpyridinium (produit de désactivation du système catalytique)
- remplacer l'acidité perdue.

Si, par ledit traitement, une fraction importante du cobalt désactivé est susceptible d'être réactivé, une fraction importante de la pyridine est ainsi éliminée du système de carbonylation (première et/ou deuxième étape). Cette perte chimique devra alors être compensée par un apport de pyridine "neuve" au système.

Cette perte paraît difficilement acceptable à l'échelle industrielle, quand on constate, au surplus, que dans la deuxième étape (conversion du pentène-3 oate d'alkyle) elle correspondrait sensiblement à la stoechiométrie en cobalt utilisée et quand on admet que des pertes physiques de pyridine (ou de base hétérocyclique azotée) inévitables à l'échelle industrielle lors des divers traitements (extraction, distillation, purges...) viendront s'y ajouter.

Aussi est-il vivement souhaitable de pouvoir disposer d'un procédé de préparation d'adipates d'alkyle comprenant la réaction de l 'oxyde de carbone et d'un alcool sur un pentènoate d'alkyle dans lequel la perte chimique de la base hétérocyclique azotée serait sensiblement diminuée, tout en maintenant sensiblement les autres performances (activité, sélectivité en adipate...).

La présente invention a donc pour premier objet un procédé de préparation d'adipates d'alkyle par réaction de l'oxyde de carbone et d'un alcool sur un pentènoate d'alkyle, sous pression supérieure à la pression atmosphérique et à température élevée, en présence d'une quantité catalytiquement efficace de cobalt ou d'un composé du cobalt et

d'une base hétérocyclique azotée, caractérisé en ce qu'on engage à la réaction un mélange renfermant essentiellement du pentène-3 oate et du pentène-2 oate d'alkyle.

La présente invention a également pour objet un procédé de préparation d'adipates d'alkyle comprenant une première étape par laquelle du butadiène est transformé de manière en soi connue, en pentène-3 oate d'alkyle, par réaction avec de l'oxyde de carbone et un alcool, sous pression supérieure à la pression atmosphérique et à température élevée, en présence d'une quantité catalytiquement efficace d'un catalyseur à base de cobalt ou de palladium, une seconde étape par laquelle ledit pentènoate est mis à réagir sur de l'oxyde de carbone et un alcool, sous pression supérieure à la pression atmosphérique et à température élevée, en présence d'une quantité catalytiquement efficace de cobalt ou d'un composé du cobalt et d'une base hétérocyclique azotée, de manière en soi connue, caractérisé en ce que

a) le mélange réactionnel issu de la première étape et, dont on a le cas échéant éliminé les matières premières en excès, les produits lourds et tout ou partie du système catalytique, est soumis à une opération d'enrichissement en pentène-2 oate d'alkyle, et en ce que
b) le mélange ainsi enrichi en pentène-2 oate d'alkyle est engagé à la seconde étape.

Selon l'invention prise dans son premier aspect, on engage à la réaction dite de seconde étape un mélange renfermant essentiellement du pentène-3 oate d'alkyle et du pentène-2 oate d'alkyle.

Bien entendu ledit mélange peut renfermer également du pentène-4 oate d'alkyle et divers autres constituants tels du pentanoate d'alkyle, de la pyridine (ou de la base hétérocyclique azotée), du méthylglutarate d'alkyle, un ou des diluants et/ou solvants organiques, des quantités variables du système catalytique mis en oeuvre lors de l'étape de réaction par laquelle le butadiène ou un mélange renfermant du butadiène est converti en pentène-3 oate d'alkyle de manière en soi connue (première étape) dès lors que ce système catalytique est compatible avec les conditions de mise en oeuvre de la dite seconde étape. Il apparaîtra clairement à l'homme de l'art que, lorsque la première étape implique la mise en oeuvre d'un système catalytique à base de cobalt et d'une base hétérocyclique azotée, il est préférable de décobalter le mélange réactionnel issu de cette première étape.

Pour une bonne mise en oeuvre de l'invention les pentènoates d'alkyle représenteront au moins 20 % en poids et, de préférence, au moins 40 % en poids du mélange engagé dans la seconde étape.

Selon la présente invention il est essentiel que du pentène-2 oate d'alkyle soit présent dans le mélange engagé à ladite seconde étape. En effet, il a maintenant été trouvé, par la Demanderesse, que cette présence a une influence remarquable à la fois sur la productivité en adipate d'alkyle recherché et sur la perte chimique en base hétérocyclique azotée. Ces effets remarquables sont sensibles dès que la teneur en pentène-2 oate d'alkyle dans le mélange (pentène-3 oate + pentène-2 oate d'alkyle) engagé représente au moins 5 % et de préférence, au moins 10 %.

Pour une bonne mise en oeuvre de la présente invention cette teneur sera au moins égale à 30 %, le maximum n'étant dicté que par des considérations d'ordre économique et/ou la disponiblité des pentène-2 oates d'alkyle.

Comme cela apparaîtra clairement à l'homme de l'art, les pentène-3 oates d'alkyle obtenus lors de ladite première étape peuvent représenter de l'ordre de 90 à 95 % (en poids) des produits identifiés formés. Aussi conviendra-t-il pour la mise en oeuvre de la présente invention, d'enrichir la matière première engagée à ladite seconde étape en pentène-2 oates d'alkyle.

Les pentène-2 oates d'alkyle se formant lors de la seconde étape, un premier mode d'enrichissement de ladite matière première en pentène-2 oate d'alkyle comprendra le recyclage au moins partiel du pentène-2 oate d'alkyle formé dans ladite seconde étape dans laquelle on aura volontairement limité le taux de conversion des pentènoates.

En pratique, le recyclage d'un mélange de pentène-2 oate et de pentène-3 oate sera avantageusement retenu, compte-tenu des difficultés associées à leur séparation.

Un second mode d'enrichissement de ladite matière première en pentène-2 oate d'alkyle comprendra le traitement préalable de tout ou partie du pentène-3 oate d'alkyle issu de ladite première étape et/ou non transformé dans ladite seconde étape, en vue de l'isomériser en un mélange enrichi en pentène-2 oate d'alkyle, ledit mélange étant alors engagé à ladite seconde étape.

Bien entendu, dans le cadre de la présente invention, il est possible d'utiliser simultanément les 2 modes d'enrichissement évoqués ci-avant et en particulier, de recycler tout ou partie du pentène-2 oate d'alkyle et de ne traiter que la partie de pentène-3 oate d'alkyle non convertie lors de ladite deuxième-étape, lorsqu'on choisit de travailler à taux de transformation partiel de cette matière dans ladite seconde étape.

Le traitement d'isomérisation peut être réalisé de diverses manières connues en elles-mêmes et en particulier, selon le procédé décrit dans le brevet américain n° 4 339 597.

Dans le cadre du second mode d'enrichissement, la Demanderesse préconise toutefois de traiter le pentène-3 oate d'alkyle, en présence de cobalt ou d'un composé du cobalt, le cas échéant d'oxyde de carbone et/ou d'une base hétérocyclique azotée, à une température supérieure ou égale à 150°C, en phase liquide, sous une pression supérieure à la pression atmosphérique, pendant un temps suffisant pour obtenir l'enrichissement souhaité.

Pour une bonne mise en oeuvre du traitement, selon la présente invention, la température de réaction sera inférieure ou égale à 250°C et de préférence comprise entre 170 et 200°C ; la pression totale en température sera supérieure ou égale à 20 bar et de préférence inférieure ou égale à 300 bar.

Pour une bonne mise en oeuvre dudit traitement, le milieu réactionnel sera, le plus souvent et sensiblement, exempt d'alcanol et pourra renfermer un diluant ou solvant organique aprotique apolaire.

A titre d'exemple de solvants organiques susceptibles de convenir à la mise en oeuvre du présent traitement on peut citer : les hydrocarbures acycliques insaturés ou, de préférence, saturés, les hydrocarbures aromatiques, les éthers tels le tétrahydrofuranne et le dioxanne.

Lorsqu'on recourt à un solvant ou diluant et, pour une bonne mise en oeuvre de l'invention, sa quantité sera telle que la teneur pondérale du pentène-3 oate d'alkyle soit d'au moins 10 % et, de préférence d'au moins 50 %.

La présence d'une base hétérocyclique azotée n'est pas nuisible dès lors qu'elle est présente en faible quantité telle que le rapport N/Co soit inférieur ou égal à 25.

Par base hétérocyclique azotée on entend ici, comme pour la première et la seconde étape, des composés bien connus de l'homme de l'art et plus particulièrement décrits notamment dans le brevet européen n° 80 957.

A titre d'exemples de tels composés on peut citer la pyridine et l'isoquinoléïne.

La durée du traitement (ou temps de séjour) peut varier dans de larges limites et dépendra pour une large part de la nature précise du système catalytique et des autres conditions opératoires retenues (présence ou non de CO, pression, température ...).

Selon un premier mode de réalisation, on met en contact le pentène-3 oate d'alkyle avec de l'oxyde de carbone en présence de cobalt carbonyle, par exemple de dicobaltoctacarbonyle.

On utilise de l'oxyde de carbone sensiblement pur, tel qu'il se présente dans le commerce. la présence d'hydrogène en faible proportion (< 1 % vol) tend à stabiliser le système catalytique. Au-dessus d'un seuil de l'ordre de 1 % (vol) une réaction d'hydrogénation parasite de la matière première est susceptible de se produire au détriment de la réaction d'isomérisation souhaitée.

Dans le cadre de ce premier mode de réalisation, le milieu réactionnel sera sensiblement exempt d'alcanol.

Selon un second mode de réalisation, on traite le pentène-3 oate d'alkyle en présence d'un carboxylate de cobalt, par exemple l'acétate de cobalt, et d'une base hétérocyclique azotée, en l'absence d'oxyde de carbone, le rapport atomique N/Co étant avantageusement choisi de telle sorte qu'il soit compatible avec le bon déroulement de la seconde étape et compris entre 2 et 25 (environ).

Dans le cadre de ce dernier mode de réalisation, le milieu réactionnel pourra renfermer avantageusement, un alcanol.

Dans les deux modes de réalisation, la concentration en cobalt du milieu réactionnel sera d'au moins 0,05 mol/l et, elle sera avantageusement comprise entre 0,1 et 2 mol/l.

En fin de traitement ou de la durée impartie à celui-ci, on détend et, le cas échéant, on refroidit.

Le mélange ainsi obtenu peut être engagé directement ou après élimination du solvant ou diluant à la réaction dite de seconde étape, par laquelle, après un ajustement de la quantité de base hétérocyclique azotée, le cas échéant de la concentration en cobalt, et ajout d'un alcanol, on produit de manière en soi connue l'adipate d'alkyle.

Les conditions de mise en oeuvre de ladite seconde étape sont largement décrites dans l'art antérieur précédemment cité, auquel on se référera pour de plus amples informations.

Brièvement, dans le cadre de cette seconde étape, on met à réagir en phase liquide, de l'oxyde de carbone et un alcool sur le mélange renfermant essentiellement du pentène-3 oate et du pentène-2 oate d'alkyle en présence de cobalt ou d'un composé du cobalt et d'une base hétérocyclique azotée ; la température de réaction est généralement comprise entre 100 et 200°C et, de préférence entre 130 et 180°C ; la pression totale en température est généralement supérieure à 50 bar et, de préférence comprise entre 100 et 300 bar. La concentration en colbalt peut varier dans de larges limites par exemple, entre 0,05 et 8 mol/l et, de préférence entre 0,1 et 2 mol/l. Le rapport molaire entre la base hétérocyclique azotée et le cobalt est généralement compris entre 0,5 et 50 et, de préférence entre 2 et 25.

On utilise de préférence un alcanol en $C_1$-$C_4$ et plus particulièrement le méthanol.

Dans le cadre de la présente invention, le mélange alimenté dans ladite seconde étape présente typiquement la composition pondérale suivante :

- pentène-3 oate d'alkyle 5 - 92 %
- pentène-2 oate d'alkyle 5 - 90 %
- pentène-4 oate d'alkyle 1 à 3 %
- pentanoate d'alkyle 1 à 10 %
- adipate de dialkyle ≃ 1 %

En fin de réaction ou du temps imparti à celle-ci, on récupère l'adipate d'alkyle par tout moyen approprié comprenant par exemple, le décobaltage du milieu réactionnel, suivi de la distillation des produits et, le cas échéant, le

recyclage des esters pentèniques n'ayant pas réagi ou par dimixtion après addition d'un solvant aprotique apolaire suivie du traitement des 2 phases obtenues, de manière en soi connue.

Les exemples suivants illustrent l'invention dans ses divers aspects.

**EXEMPLES 1 à 3 ;** essais témoins (a) et (b) :

Ces exemples concernent l'alcoxycarbonylation des pentènoates d'alkyles.

Mode opératoire :

Dans un autoclave en acier inoxydable de 300 ml de capacité, muni d'une agitation magnétique centrale, on charge :

| | |
|---|---|
| du (ou des) pentènoate(s) de méthyle | 0,58 mol |
| du dicobaltoctacarbonyle | 0,012 mol |
| de la pyridine | 0,115 mol |
| du méthanol | 1,16 mol |
| et du butylbenzène (étalon interne de CPG) | 5 g. |

L'agitation (1200 t/min) est mise en route, l'autoclave est purgé par du CO (3x 50 bar) puis pressurisé à 150 bar par le CO (contenant 0,5 % vol d'$H_2$) et porté à 170°C. L'autoclave est maintenu dans ces conditions (170°C, 150 bar) pendant toute la durée de l'essai. Un balayage continu du ciel gazeux est assuré par un débit d'entrée de CO constant, fixé à 60 l/h (CNTP).

Des prélèvements de la masse réactionnelle sont effectués à intervalles réguliers. Après dilution par le méthanol (qsp 15 ml) ils sont analysés par CPG et chromatographie liquide d'appariement d'ions.

Les conventions suivantes sont utilisées :

P3 : pentène-3 oate de méthyle
P2 : pentène-2 oate de méthyle
P4 : pentène-4 oate de méthyle
Pa : pentanoate de méthyle
E1 : adipate de diméthyle
E2 : méthyl-2 glutarate de diméthyle
E3 : éthylsuccinate de diméthyle
E4 : propylmalonate de diméthyle

$$Ei = E1 + E2 + E3 + E4$$

$$L = \frac{E1}{Ei}$$

$$Pi = P2 + P3 + P4$$

Py = pyridine

$$TT = \frac{\text{mmol substrat engagées - mmol substrat dosées}}{\text{mmol substrat engagées}} \times 100$$

$$RR = \frac{\text{mmol produit dosées}}{\text{mmol substrat engagées}} \times 100$$

$$RT = RR/TT = \text{sélectivité}$$

$$k = -\,Ln(1\text{-}TT)/t \text{ en } h^{-1}$$

C (PY) = nombre de kg de pyridine dégradée par tonne d'acide adipique potentielle déterminé avec les 2 hypothèses suivantes :

- réacteur batch avec TT de 90 %
- taux d'hydrolyse de l'adipate de diméthyle en acide adipique = 100 %

L'essai témoin (a) est réalisé au départ de pentène-3 oate de méthyle.

L'essai témoin (b) est réalisé au départ de pentène-2 oate de méthyle.

Les exemples 1 à 3 sont réalisés au départ d'un mélange de pentène-3 oate et de pentène-2 oate, dont la teneur en pentène-2 oate figure au tableau (I) ci-après dans lequel sont également indiqués les résultats obtenus, t indiquant le temps nécessaire pour obtenir un taux de transformation total du(ou des) pentèneoates(s).

TABLEAU I

| Réf | %P2 | t(mn) | k(h-1) | RT % | | L % | C(Py) |
|---|---|---|---|---|---|---|---|
| | | | | Pa | E1 | | |
| a | 0 | 220 | 1,3 | 9 | 67 | 82 | 10 |
| 1 | 5 | 200 | 1,5 | 9 | 69 | 82 | 9 |
| 2 | 10 | 180 | 1,5 | 9 | 73 | 82 | 9 |
| 3 | 50 | 155 | 1,6 | 9 | 70 | 82 | 8 |
| b | 100 | 180 | 2 | 8 | 72 | 80 | 5 |

EXEMPLES 4 et 5 :

Ces exemples illustrent l'isomérisation du pentène-3 oate de méthyle.

Dans l'autoclave et selon le mode opératoire décrit pour l'exemple 1 ci-avant on réalise un essai sur une charge renfermant :

0,58 mol de pentène-3 oate de méthyle
0,012 mol de dicobalt octacarbonyle

0,115 mol de pyridine ┐ Exemple 4
46 ml de toluène ┘
61 ml de toluène ┐ Exemple 5
(absence de pyridine) ┘

Dans les deux exemples la température de réaction est de 170°C la pression est de 150 bar, la vitesse d'agitation est de 1200 tour/min et le débit d'entrée de l'oxyde de carbone est de 60 l/h (CNTP).

Les résultats obtenus sont consignés dans le tableau III ci-après, dans lequel T désigne la durée de réaction.

TABLEAU III

| Réf | Py (mol) | T(h) | TT(P3) (%) | RT % | | | |
|---|---|---|---|---|---|---|---|
| | | | | P2 | P4 | Pa | Ei |
| 4 | 0,115 | 2 | 31 | 70,6 | 6,3 | 12,9 | 2,2 |
| 5 | 0 | 3 | 56,5 | 74,2 | 6,8 | 7,7 | 3,0 |

EXEMPLES 6 et 7 :

Ces exemples illustrent l'isomérisation du pentène-3 oate de méthyle.

Dans un autoclave de 125 ml de capacité en Hastelloy® B2, on charge :

0,1 mol de pentène-3 oate de méthyle
0,004 mol d'acétate de cobalt
0,02 mol de pyridine
8 ml de méthanol (Exemple 6)
ou 10 ml de toluène (Exemple 7)

L'autoclave est fermé, purgé par de l'argon et porté à 200°C sous agitation (par secousses). Après 5 heures de réaction à cette température, il est refroidi. La masse réactionnelle est soutirée, diluée par du méthanol et analysée par chromatographie en phase gazeuse.

Les résultats obtenus sont consignés dans le tableau IV, ci-après :

TABLEAU IV

| Réf | Solvant | TT(P3) (%) | RT % | | |
|---|---|---|---|---|---|
| | | | P2 | P4 | Pa |
| 6 | Méthanol | 50 | 68 | 1,6 | 6 |
| 7 | Toluène | 21 | 30 | 3 | 8 |

EXEMPLE 8 :

Cet exemple illustre un enchaînement isomérisation - alcoxycarbonylation -

Dans un autoclave en acier inoxydable de 300 ml de capacité, muni d'une agitation magnétique centrale, on charge :

0,58 mol de pentène-3 oate de méthyle
0,012 mol de dicobaltoctacarbonyle
61 ml de toluène
5 g de n-butylbenzène (étalon interne).

La réaction est conduite pendant 90 mn dans les conditions de l'exemple 1 ci-avant. L'autoclave est alors refroidi et dépressurisé. L'analyse d'une fraction aliquote de la masse réactionnelle conduit aux résultats suivants :

TT P3 = 43 %
RT P2 = 77 %
RT P4 = 8,3 %
RT Pa = 8,2 %

La masse réactionnelle renferme notamment :

- 0,33 mol de P3 (61 %)
- 0,19 mol de P2 (35 %)
- 0,02 mol de P4 ( 4 %)

normalisation à 100 % des Pi

A cette masse réactionnelle laissée dans l'autoclave, on ajoute :

- du méthanol (26 ml ; 0,638 mol)
- de la pyridine (12 ml ; 0,148 mol)

L'autoclave est alors fermé, purgé par de l'oxyde de carbone (3 x 10 bar) puis porté à 170°C sous 150 bar de CO [contenant 0,5 % (vol) d'hydrogène].

La vitesse d'agitation est de 1200 t/mn ;

L'oxyde de carbone est alimenté à débit constant de 60 l/h (CNTP).

Après 140 mn de réaction, l'autoclave est refroidi et dégazé. La masse réactionnelle est alors soutirée et analysée par chromatographie en phase gazeuse et chromatographie liquide d'appariement d'ions.

Les résultats sont les suivants :

TT(Pi) = 99 %

k = 2 $h^{-1}$

| | | |
|---|---|---|
| RT ($E_1$) | = 70 % | par rapport aux Pi du brut réactionnel de l'étape d'isomérisation |
| RT ($E_2$) | = 11 % | |
| RT ($E_3$) | = 4 % | |
| RT (Pa) | = 8 % | |

C (Py) = 4

## Revendications

1. Procédé de préparation d'adipates d'alkyle par réaction de l'oxyde de carbone et d'un alcool sur un penténoate d'alkyle, sous pression supérieure à la pression atmosphérique et à température élevée, en présence d'une quantité catalytiquement efficace de cobalt ou d'un composé du cobalt et d'une base hétérocyclique azotée, caractérisé en ce qu'on engage à la réaction un mélange renfermant essentiellement du pentène-3 oate et du pentène-2 oate d'alkyle, le pentène-2 oate d'alkyle représentant au moins 5 % dudit mélange.

2. Procédé de préparation d'adipates d'alkyle comprenant une première étape par laquelle du butadiène est transformé de manière en soi connue, en pentène-3 oate d'alkyle, par réaction avec de l'oxyde de carbone et d'un alcool, sous pression supérieure à la pression atmosphérique et à température élevée, en présence d'une quantité catalytiquement efficace d'un catalyseur à base de cobalt ou de palladium, une seconde étape par laquelle ledit penténoate est mis à réagir de manière en soi connue, sur de l'oxyde de carbone et un alcool, sous pression supérieure à la pression atmosphérique et à température élevée, en présence d'une quantité catalytiquement efficace de cobalt ou d'un composé du cobalt et d'une base hétérocyclique azotée, caractérisé en ce que

   a) le mélange réactionnel issu de la première étape et, dont on a le cas échéant éliminé les matières premières en excès, les produits lourds et tout ou partie du système catalytique, est soumis à une opération d'enrichissement en pentène-2 oate d'alkyle, et en ce que
   b) le mélange ainsi enrichi en pentène-2 oate d'alkyle dont la teneur totale en penténoates d'alkyle est de au moins 20% en poids, le pentène-2 oate représentant au moins 5% dans le mélange de pentène-3 et pentène-2-oates d'alkyle, est engagé à la seconde étape.

3. Procédé selon la revendication 2, caractérisé en ce que l'opération d'enrichissement en pentène-2 oate d'alkyle est réalisée pour tout ou partie par le recyclage d'au moins une partie du pentène-2 oate d'alkyle formé dans ladite seconde étape dans laquelle on aura volontairement limité le taux de conversion des pentèneoates.

4. Procédé selon la revendication 3, caractérisé en ce que ledit enrichissement est réalisé pour tout ou partie par le recyclage d'un mélange de pentène-2 oate et de pentène-3 oate non convertis dans la seconde étape.

5. Procédé selon la revendication 2, cartactérisé en ce que l'opération d'enrichissement en pentène-2 oate d'alkyle est réalisée pour tout ou partie par un traitement préalable d'isomérisation du pentène-3 oate d'alkyle issu de ladite première étape et/ou non transformé dans ladite seconde étape.

6. Procédé selon la revendication 5, caractérisé en ce que l'isomérisation est réalisée par mise en contact du pentène-3 oate d'alkyle et de cobalt ou d'un composé du cobalt, le cas échéant d'oxyde de carbone et/ou d'une base hétérocyclique azotée, à une température supérieure ou égale à 150°C, en phase liquide et sous une pression supérieure à la pression atmosphérique.

7. Procédé selon la revendication 6, caractérisé en ce que la température de réaction est comprise entre 170 et 200°C.

8. Procédé selon la revendication 6 ou 7, caractérisé en ce que la pression totale en température est supérieure ou égale à 20 bar.

9. Procédé selon l'une quelconque des revendications 6 à 8, caractérisé en ce que la pression totale en température est inférieure ou égale à 300 bar.

10. Procédé selon l'une quelconque des revendications précédentes, caractérisé en ce que le pentène-2 oate d'alkyle représente au moins 10 % dans le mélange de pentène-3 oate et de pentène-2 oate d'alkyle engagé à la seconde étape.

**Claims**

1. Process for the preparation of alkyl adipates by reaction of carbon monoxide and of an alcohol with an alkyl pentenoate at a pressure above atmospheric pressure and at elevated temperature, in the presence of a catalytically effective quantity of cobalt or of a cobalt compound and of a nitrogenous heterocyclic base, characterized in that a mixture containing essentially alkyl 3-pentenoate and 2-pentenoate is introduced in the reaction, the alkyl 2-pentenoate representing at least 5 % of the said mixture.

2. Process for the preparation of alkyl adipates comprising a first stage by which butadiene is converted in a manner known per se to alkyl 3-pentenoate by reaction with carbon monoxide and an alcohol, at a pressure above atmospheric pressure and at elevated temperature, in the presence of a catalytically effective quantity of a catalyst based on cobalt or palladium, a second stage by which the said pentenoate is reacted in a manner known per se with carbon monoxide and an alcohol, at a pressure above atmospheric pressure and at elevated temperature, in the presence of a catalytically effective quantity of cobalt or of a cobalt compound and of a nitrogenous heterocyclic base, characterized in that

    a) the reaction mixture originating from the first stage and from which the excess raw materials, the heavy products and all or part of the catalyst system have been removed if appropriate, is subjected to an operation of enrichment in alkyl 2-pentenoate, and in that
    b) the mixture thus enriched in alkyl 2-pentenoate, the total alkyl pentenoate content of which is at least 20 % by weight, the 2-pentenoate representing at least 5 % in the mixture of alkyl 3- and 2-pentenoates, is introduced in the second stage.

3. Process according to Claim 2, characterized in that the operation of enrichment in alkyl 2-pentenoate is carried out wholly or partially by recycling at least part of the alkyl 2-pentenoate formed in the said second stage, in which the degree of conversion of the pentenoates will have been deliberately limited.

4. Process according to Claim 3, characterized in that the said enrichment is carried out wholly or partially by recycling a mixture of 2-pentenoate and and 3-pentenoate which are not converted in the second stage.

5. Process according to Claim 2, characterized in that the operation of enrichment in alkyl 2-pentenoate is carried out wholly or partially by a preliminary isomerization treatment of the alkyl 3-pentenoate originating from the said first stage and/or not converted in the said second stage.

6. Process according to Claim 5, characterized in that the isomerization is carried out by bringing alkyl 3-pentenoate into contact with cobalt or a cobalt compound, if appropriate carbon monoxide and/or a nitrogenous heterocyclic base, at a temperature higher than or equal to 150°C, in liquid phase and at a pressure above atmospheric pressure.

7. Process according to Claim 6, characterized in that the reaction temperature is between 170 and 200°C.

8. Process according to Claim 6 or 7, characterized in that the total pressure at temperature is higher than or equal to 20 bar.

9. Process according to any one of Claims 6 to 8, characterized in that the total pressure at temperature is lower than or equal to 300 bar.

10. Process according to any one of the preceding claims, characterized in that the alkyl 2-pentenoate represents at least 10 % in the mixture of alkyl 3-pentenoate and 2-pentenoate introduced in the second stage.

**Patentansprüche**

1. Verfahren zur Herstellung von Alkyladipaten durch Umsetzung eines Kohlenstoffoxids und eines Alkohols mit einem Alkylpentenat bei über dem Atmosphärendruck liegendem Druck und bei erhöhter Temperatur in Gegenwart einer katalytisch wirksamen Menge an Kobalt oder einer Kobaltverbindung und einer heterocyclischen Stickstoffbase, dadurch **gekennzeichnet,** daß man bei der Reaktion ein Gemisch, umfassend im wesentlichen Alkylpent-3-enat und Alkylpent-2-enat, einsetzt, wobei das Alkylpent-2-enat mindestens 5 % des Gemisches ausmacht.

2. Verfahren zur Herstellung von Alkyladipaten, umfassend eine erste Stufe, in der man Butadien in an sich bekannter Weise durch Umsetzung mit dem Kohlenstoffoxid und einem Alkohol bei über dem Atmosphärendruck liegendem Druck und bei erhöhter Temperatur in Gegenwart einer katalytisch wirksamen Menge eines Katalysators auf der Basis von Kobalt oder Palladium in Alkylpent-3-enat umwandelt, eine zweite Stufe, in der man das Pentenat in an sich bekannter Weise mit dem Kohlenstoffoxid und einem Alkohol bei über dem Atmosphärendruck liegendem Druck und bei erhöhter Temperatur in Gegenwart einer katalytisch wirksamen Menge an Kobalt oder einer Kobaltverbindung und einer heterocyclischen Stickstoffbase umsetzt, dadurch **gekennzeichnet,** daß man

   a) das aus der ersten Stufe hervorgehende Reaktionsgemisch, von dem man gegebenenfalls überschüssige Ausgangsmaterialien, schwere Verbindungen und das vollständige katalytische System oder einen Teil davon entfernt hat, einem Arbeitsschritt zur Anreicherung von Alkylpent-2-enat unterwirft, und
   b) das so an Alkylpent-2-enat angereicherte Gemisch, dessen Gesamtgehalt an Alkylpentenaten mindestens 20 Gew.-% beträgt, wobei das Pent-2-enat mindestens 5 Gew.-% in dem Gemisch aus Alkylpent-3-enaten und Alkylpent-2-enaten ausmacht, in der zweiten Stufe einsetzt.

3. Verfahren nach Anspruch 2, dadurch **gekennzeichnet,** daß man den Arbeitsschritt der Anreicherung an Alkylpent-2-enat vollständig oder teilweise durch Zurückführen mindestens eines Teils des in der zweiten Stufe gebildeten Alkylpent-2-enats durchführt, in der man freiwillig den Umwandlungsgrad der Pentenate beschränkt hat.

4. Verfahren nach Anspruch 3, dadurch **gekennzeichnet,** daß man die Anreicherung vollständig oder teilweise durch Zurückführen eines Gemisches aus in der zweiten Stufe nicht umgewandeltem Alkylpent-2-enat und Alkylpent-3-enat durchführt.

5. Verfahren nach Anspruch 2, dadurch **gekennzeichnet,** daß man den Arbeitsschritt der Anreicherung an Alkylpent-2-enat vollständig oder teilweise durch eine vorherige Isomerisierungsbehandlung des aus der ersten Stufe hervorgegangenen und/oder in der zweiten Stufe nicht umgewandelten Alkylpent-3-enats durchführt.

6. Verfahren nach Anspruch 5, dadurch **gekennzeichnet,** daß man die Isomerisierung durch In-Kontaktbringen des Alkylpent-3-enats und Kobalt oder einer Kobaltverbindung, gegebenenfalls Kohlenstoffoxid und/oder einer heterocyclischen Stickstoffbase bei einer Temperatur größer oder gleich 150°C in flüssiger Phase und unter einem Druck, der über dem Atmosphärendruck liegt, durchführt.

7. Verfahren nach Anspruch 6, dadurch **gekennzeichnet,** daß die Reaktionstemperatur zwischen 170 und 200°C liegt.

8. Verfahren nach Anspruch 6 oder 7, dadurch **gekennzeichnet,** daß der gesamte Druck bei der Temperatur größer oder gleich 20 bar ist.

9. Verfahren nach einem der Ansprüche 6 bis 8, dadurch **gekennzeichnet,** daß der gesamte Druck bei der Temperatur kleiner oder gleich 300 bar ist.

10. Verfahren nach einem der vorausgehenden Ansprüche, dadurch **gekennzeichnet,** daß das Alkylpent-2-enat mindestens 10 % in dem Gemisch aus Alkylpent-3-enat und Alkylpent-2-enat, das in der zweiten Stufe eingesetzt wurde, ausmacht.